(19) Europäisches Patentamt
European Patent Office
Office européen des brevets

(11) EP 1 647 274 A1

(12) **EUROPEAN PATENT APPLICATION**
published in accordance with Art. 158(3) EPC

(43) Date of publication:
**19.04.2006 Bulletin 2006/16**

(21) Application number: **04747906.8**

(22) Date of filing: **16.07.2004**

(51) Int Cl.:
***A61K 31/451*** *(2000.01)* ***A61K 9/06*** *(1968.09)*
***A61K 9/08*** *(1974.07)* ***A61K 45/00*** *(1985.01)*
***A61P 17/04*** *(2000.01)* ***A61P 27/02*** *(2000.01)*
*C07D 211/64 (1985.01)*

(86) International application number:
**PCT/JP2004/010542**

(87) International publication number:
**WO 2005/007161 (27.01.2005 Gazette 2005/04)**

(84) Designated Contracting States:
**AT BE BG CH CY CZ DE DK EE ES FI FR GB GR HU IE IT LI LU MC NL PL PT RO SE SI SK TR**

(30) Priority: **17.07.2003 JP 2003275845**

(71) Applicant: **ONO PHARMACEUTICAL CO., LTD.**
**Osaka-shi 541-8526 (JP)**

(72) Inventors:
- **SHII, Daisuke,**
**c/o SANTEN PHARMACEUTICAL CO., LTD.**
**Ikoma-shi, Nara 6300101 (JP)**
- **ODA, Tomoko,**
**c/o SANTEN PHARMACEUTICAL CO., LTD.**
**Ikoma-shi, Nara 6300101 (JP)**
- **MIYAKE, Hideki,**
**c/o SANTEN PHARMACEUTICAL CO., LTD**
**Ikoma-shi, Nara 6300101 (JP)**
- **FUJITA, Manabu,**
**c/o ONO PHARMACEUTICAL CO., LTD.**
**Mishima-gun, Osaka 6188585 (JP)**

(74) Representative: **Peaucelle, Chantal et al**
**Cabinet Armengaud Aîné**
**3, Avenue Bugeaud**
**75116 Paris (FR)**

# (54) REMEDY FOR PRURITUS COMPRISING PIPERIDINE DERIVATIVE AS THE ACTIVE INGREDIENT

(57) It is intended to find out a novel pharmacological effect of a piperidine derivative (I). Because of having an excellent antipruritic effect, the piperidine derivative (1) is useful as a therapeutic agent for itching of any types such as eye itching, skin itching and systemic itching.

EP 1 647 274 A1

## Description

Technical field

The present invention relates to a therapeutic agent for pruritus comprising a piperidine derivative as an active ingredient.

Background art

When itching receptors existing in epidermis-dermis junction of skin or mucosa are stimulated by transmitters (pruritus-inducing substances) and the stimulation is transmitted to central nerve, the itching is recognized. Well-known pruritus-inducing transmitters include, for example, histamine, kinin, bile salt, substance P, prostaglandin and so on. It is guessed that itching owing to allergic factors is involved in transmitters such as histamine etc. released from mast cells etc. It is known that antihistaminic drugs exhibit stronger effects than antiallergic drugs.

It is noted that itching includes, for example, eye itching, skin itching, ear itching, nose itching, systemic itching occurring in human beings or animals. Eye itching is a disease which makes eyes, eyelids, lid margins or the like, itchy because of pollens, dusts, ticks, fungi, hairs of pets, contact lenses, cosmetics *etc.* In addition, conjunctivae congests by scratching eyes and conjunctival papillae redden and grow. When it becomes serious, lesion occurs in corneas and scleras to happen to proceed to vernal conjunctivitis.

WO 02/14280 relates to piperidine derivatives having PDE4 inhibitory activities. In the specification, it is described that these piperidine derivatives are useful for treating inflammatory disease such as nephritis etc., allergic disease such as allergic rhinitis etc., autoimmune disease and so on. However, it is not described at all that piperidine derivatives described in the above-mentioned WO 02/14280 are useful for treating itching which is the most major symptom due to allergic disease.

DISCLOSURE OF THE INVENTION

Though it is noted that the piperidine derivatives described in the above-mentioned WO02/14280 have various pharmacological effects, it is an interesting subject to find additional effectiveness on itching as a new pharmacological effect.

Conducting intensive studies to accomplish the above-mentioned purpose, the present inventors found out that the piperidine derivatives represented by formula (I) exhibit an excellent antipruritic effect and got to the present invention. The present inventors also found out that {4-cyano-4-[3-(cyclopentyloxy)-4-(difluoromethoxy)phenyl]piperidine-1-yl}acetic acid or a solvate thereof among the piperidine derivatives represented by formula (I) has an excellent inhibitory effect on especially eye itching and completed the present invention. That is, the present invention relates to

(1) Therapeutic agent for pruritus comprising a compound represented by formula (I)

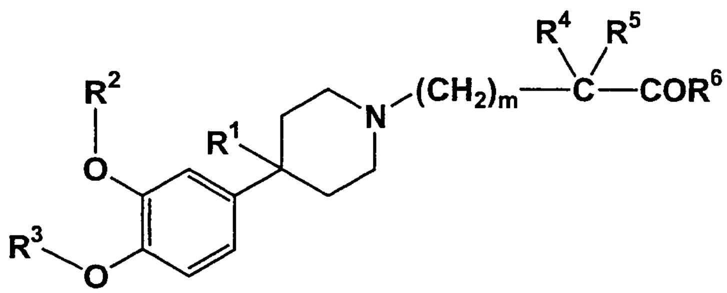

(I)

(wherein, $R^1$ is 1) a hydrogen atom, or 2) cyano, $R^2$ and $R^3$ are each independently 1) C1-8 alkyl, 2) C3-7 cycloalkyl, 3) C1-8 alkyl substituted by C3-7 cycloalkyl, 4) C1-8 alkyl substituted by 1, 2 or 3 halogen atom(s), 5) a hydrogen atom, 6) C1-8 alkyl substituted by phenyl, 7) C1-8 alkyl substituted by C1-8 alkoxy, or 8)

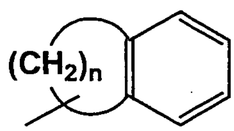

(wherein, n is 1 to 5.), $R^4$ and $R^5$ are each independently 1) a hydrogen atom, or 2) C1-8 alkyl, or $R^4$ and $R^5$ form a C3-7 saturated carbocyclic ring together with their binding carbon atom, $R^6$ is 1) hydroxyl, 2) C1-8 alkoxy, 3) -NHOH, or 4) C1-8 alkoxy substituted by phenyl, m is 0 or an integer of 1 to 4.),
a salt thereof, or a solvate thereof as the active ingredient,
(2) The therapeutic agent for pruritus according to the above-mentioned (1), wherein the compound is {4-cyano-4-[3-(cyclopentyloxy)-4-(difluoromethoxy)phenyl]piperidine-1-yl}acetic acid,
(3) The therapeutic agent for pruritus according to the above-mentioned (2), wherein the active ingredient is {4-cyano-4-[3-(cyclopentyloxy)-4-(difluoromethoxy)phenyl]piperidine-1-yl}acetic acid monohydrate,
(4) The therapeutic agent for pruritus according to the above-mentioned (1), wherein the pruritus is eye itching,
(5) The therapeutic agent for pruritus according to the above-mentioned (1), wherein the therapeutic agent is in a form of a liquid preparation,
(6) The therapeutic agent for pruritus according to the above-mentioned (1), wherein the therapeutic agent is in a form of an external preparation,
(7) The therapeutic agent for pruritus according to the above-mentioned (5), wherein the liquid preparation is an eye drop,
(8) The therapeutic agent for pruritus according to the above-mentioned (6), wherein the external preparation is an ointment,
(9) The therapeutic agent for pruritus according to the above-mentioned (8), wherein the ointment is an ophthalmic ointment,
(10) The therapeutic agent for pruritus according to the above-mentioned (7), wherein the eye drop contains the active ingredient of 0.01 to 1 % (w/v),
(11) A pharmaceutical composition comprising the therapeutic agent for pruritus according to the above-mentioned (1) and at least one selected from steroidal anti-inflammatory drugs, nonsteroidal anti-inflammatory drugs, antiallergic drugs, antihistamine drugs, therapeutic agents for glaucoma, antibiotics, antibacterial drugs, antivirus drugs, and antifungal drugs,
(12) A method for treating pruritus comprising administering to a mammal an effective amount of the compound represented by formula (I) described in the above-mentioned (1), a salt thereof or a solvate thereof,
(13) Use of the compound represented by formula (I) described in the above-mentioned (1), a salt thereof or a solvate thereof for manufacturing a therapeutic agent for pruritus. In formula (I), C1-8 alkyl means methyl, ethyl, propyl, butyl, pentyl, hexyl, heptyl, octyl and an isomer thereof. C1-8 alkoxy means methoxy, ethoxy, propoxy, butoxy, pentyloxy, hexyloxy, heptyloxy, octyloxy and an isomer thereof. Halogen atom means chlorine, bromine, fluorine, iodine. C3-7 cycloalkyl means cyclopropyl, cyclobutyl, cyclopentyl, cyclohexyl and cycloheptyl. C3-7 saturated carbocyclic ring which $R^4$ and $R^5$ form together with their binding carbon atom means C3-7 cycloalkyl, *i.e.*, cyclopropyl, cyclobutyl, cyclopentyl, cyclohexyl and cycloheptyl.

Unless otherwise noted, all isomers are included in the piperidine derivatives of the present invention. For example, alkyl, alkoxy and alkylene group means straight-chain or branched-chain ones. In addition, isomers on double bond, ring, fused ring (E-, Z-, cis-, trans-isomer), isomers due to asymmetric carbon atom(s) (R-, S-isomer, α-, β-configuration, enantiomer, diastereomer), optically active isomers (D-, L-, d-, l-isomer), polar compounds obtained by chromatographic separation (more polar compound, less polar compound), equilibrium compounds, mixtures thereof at any ratios and racemic mixtures are also included in the present invention.

The salts of the compounds represented by formula (I) include all of pharmaceutically acceptable salts. As pharmaceutically acceptable salts, nontoxic, water-soluble salts are preferable. The suitable salts include, for example, salts of alkali metals (*e.g.*, potassium, sodium, lithium, *etc.),* salts of alkaline earth metals (*e.g.*, calcium, magnesium, *etc.*), ammonium salts (*e.g.*, tetramethylammonium salt, tetrabutylammonium salt, *etc.*), organic amine (*e.g.*, triethylamine, methylamine, dimethylamine, cyclopentylamine, benzylamine, phenethylamine, piperidine, monoethanolamine, diethanolamine, lysine, arginine, N-methyl-D-glucamine, *etc.*), acid addition salts (salts of inorganic acids (*e.g.*, hydrochloride salt, hydrobromide salt, hydroiodide salt, sulfate salt, phosphate salt, nitrate salt *etc.*), and salts of organic acids (*e.g.*, acetate, trifluoroacetate, lactate, tartrate, oxalate, fumarate, maleate, benzoate, citrate, methanesulfonate, ethanesulfonate, benzenesulfonate, toluenesulfonate, isethionate, glucuronate, gluconate *etc.*).

The solvates of the compounds represented by formula (I) include, for example, hydrates, solvates of the alcohols (*e.g.*, ethanol *etc.*), and so on. The solvates are preferably nontoxic and water-soluble. In addition, the solvate of the compound in the present invention includes the solvates such as, hydrates, solvates of the alcohols (*e.g.*, ethanol *etc.*) *etc.,* of the above-mentioned salts of alkali (earth) metals, ammonium salts, salts of organic amine, acid addition salts, and so on.

The compound of the present invention can be converted into the above-mentioned N-oxide or the above-mentioned solvates by known methods.

In formula (I), $R^1$ is preferably cyano. $R^2$ is preferably C1-8 alkyl, C3-7 cycloalkyl, or C1-8 alkyl substituted by

C3-7 cycloalkyl, particularly preferably methyl, ethyl, isopropyl, 2-methylpropyl, cyclobutyl, cyclopentyl or cyclopropylmethyl. $R^3$ is preferably C1-8 alkyl or C1-8 alkyl substituted by 1 to 3 halogen atom(s), particularly preferably methyl, ethyl, isopropyl, 2-methylpropyl, or difluoromethyl. $R^4$ and $R^5$ are preferably hydrogen atom. $R^6$ is preferably hydroxyl or -NHOH, particularly preferably hydroxyl. Preferred piperidine derivatives represented by formula (I) is {4-cyano-4-[3-(cyclopentyloxy)-4-(difluoromethoxy)phenyl]piperidine-1-yl}acetic acid or hydrate thereof. Preferred hydrate is {4-cyano-4-[3-(cyclopentyloxy)-4-(difluoromethoxy)phenyl]piperidine-1-yl}acetic acid monohydrate.

The compounds represented by formula (I), a salt thereof, or a solvate thereof can be prepared according to the method described in WO02/14280.

In addition, the therapeutic agent for pruritus of the present invention can be administered in combination with other pharmaceutical preparations for the purpose of 1) complement and/or enhancement of the therapeutic effect of the agents of the present invention, 2) improvement of dynamics/absorption and lowering of dose of the agents of the present invention and/or 3) alleviation of side effects of the agents of the present invention.

The therapeutic agent of the present invention and the other therapeutic agents can be administered in the form of one preparation containing both agents incorporated therein or can be administered in separate preparations respectively. In the case where the therapeutic agent of the present invention and the other therapeutic agents are administered in separate preparations, they can be administered simultaneously or at different times. Additionally, in the latter case, the agent of the present invention can be administered before the other therapeutic agents. Alternatively, the other therapeutic agents can be administered before the agent of the present invention. The administration method of these therapeutic agents can be the same or different.

The other therapeutic agents can be low-molecular compounds. In addition, they can be macromolecular proteins, polypeptides, polynucleotides (DNA, RNA, and genes), antisenses, decoys, antibodies, vaccines and so on. The dose of the other pharmaceutical preparations can be appropriately selected on the basis of clinical dose. The ratio of the agent of the present invention and the other therapeutic agents can be appropriately selected depending on the age and body weight of administering object, the administration method, the administration time, *etc.* For example, the other therapeutic agents can be used from 0.01 to 100 parts by weight to 1 part by weight of the agent of the present invention. The other therapeutic agents can be administered combining at least two agents at appropriate ratio. The other therapeutic agents which complement and/or enhance the effect of the agents of the present invention include not only ones which have been found but ones which will be found from now based on the above-mentioned mechanism.

The other therapeutic agents include, for example, steroidal anti-inflammatory drugs (*e.g.* dexamethasone, prednisolone, *etc.*), nonsteroidal anti-inflammatory drugs (*e.g.* diclofenac sodium, pranoprofen, *etc.*), antiallergic drugs (*e.g.* tranilast, ketotifen fumarate, sodium cromoglicate, *etc.*), antihistamine drugs (*e.g.* diphenhydramine hydrochloride *etc.*), therapeutic agents for glaucoma (*e.g.* pilocarpine hydrochloride, physostigmine salicylate, timolol, isopropyl unoprostone *etc.*), antibiotics (*e.g.* gentamicin sulfate, fradiomycin sulfate, tobramycin, sulbenicillin, cefmenoxime, erythromycin, colistin, oxytetracycline, polymyxin B, chloramphenicol, micronomicin, dibekacin, sisomicin *etc.*), antibacterial drugs(*e.g.* sulfamethizole, sulfamethoxazole, ofloxacin, norfloxacin, lomefloxacin hydrochloride, enoxacin, ciprofloxacin hydrochloride, cinoxacin, sparfloxacin, tosufloxacin tosylate, nalidixic acid, pipemidic acid trihydrate, pipemidic acid, fleroxacin, levofloxacin *etc.*), antivirus drugs (*e.g.* idoxuridine, acyclovir *etc.*), antifungal drugs (*e.g.* pimaricin, fluconazole, miconazole, amphotericin B, flucytosine, itraconazole *etc.*) and so on.

The piperidine derivatives of the present invention exhibit an effect on treating and inhibiting itching such as eye itching, skin itching, ear itching, nose itching, systemic itching and so on occurring in human beings and animals. More preferably they are used as a therapeutic agent for eye itching.

Eye itching in the present invention is a disease which makes eyes, eyelids, lid margins or the like, itchy because of pollens, dusts, ticks, fungi, hairs of pets, contact lenses, cosmetics, ocular injuries *etc.* In addition, eye itching includes ones which occur with various eye diseases such as allergic conjunctivitis, vernal keratoconjunctivitis, atopic keratoconjunctivitis, infectious keratoconjunctivitis, blepharitis, dry eye, conjunctivitis, corneal herpes, corneal ulcer and so on, or ophthalmic operation etc. The therapeutic agent for pruritus of the present invention exhibits excellent an inhibitory effect on itching as apparent from the result of the after-mentioned test of inhibitory effect on eye itching.

To the therapeutic agent for pruritus of the present invention, if necessary, can be added pharmaceutically acceptable additives, and a sole preparation or a formulated preparation can be prepared by widely used technique.

The therapeutic agent for pruritus of the present invention can be administered orally or parenterally. Examples of oral preparations include liquid preparations for internal use (*e.g.* elixir, syrup, pharmaceutically acceptable aqueous solution, suspension, emulsion *etc.*), solid preparations for internal use (*e.g.* tablets (including sublingual tablets, disintegrating tablets in oral cavity), pills, capsules (including hard capsules, soft capsules, gelatin capsules, micro capsules.), powders, granules, troches *etc.*) and so on. Examples of parenteral preparations include liquid preparations (*e.g.* injections (*e.g.* subcutaneous injections, intravenous injections, intramuscular injections, intraperitoneal injections, drops *etc.*), eye drops (*e.g.* aqueous eye drops (aqueous eye drop solutions, aqueous eye drop suspensions, viscous eye drops, solubilized eye drops *etc.*), nonaqueous eye drops (nonaqueous eye drop solutions, nonaqueous eye suspensions)) *etc.),* external preparations (*e.g.* ointments (ophthalmic ointments *etc.),* gels, creams, fomentations, cataplasms,

liniments *etc.*), nebulas, inhalants, sprays, nasal drops, suppositories (*e.g.* rectal suppositories, vaginal suppositories *etc.*) and so on. These preparations can be release control preparations such as quick-release preparations, sustained-release preparations and so on. These preparations can be prepared by known methods such as the method described in Japanese Pharmacopoeia and so on.

The liquid preparations for internal use as oral preparations are prepared by, for example, dissolving, suspending or emulsifying active ingredients into a usually usable diluent (*e.g.*, purified water, ethanol, mixture thereof *etc.*). These liquid agent can comprise a wetting agent, a suspending agent, an emulsifier, a sweetening agent, a flavor, an aromatic, a preservative, a buffer, *etc.*

The solid preparations for internal use as oral preparations are prepared by, for example, mixing active ingredients with an excipient (*e.g.*, lactose, mannitol, glucose, microcrystalline cellulose, starch *etc.*), a binder (*e.g.*, hydroxypropyl cellulose, polyvinyl pyrrolidone, magnesium metasilicoaluminate *etc.),* a disintegrant (*e.g.*, calcium fibrinoglycolate *etc.*), a lubricant (*e.g.*, magnesium stearate *etc.*), a stabilizer, a solubilizing agent (*e.g.*, glutamic acid, aspartic acid *etc.*) or the like, followed by carrying out conventional methods. If necessary, the solid preparations can be coated with a layer of coating agent (*e.g.*, white sugar, gelatin, hydroxypropyl cellulose, hydroxypropyl methyl cellulose phthalate *etc.*) or two or more layers.

The external preparations as parenteral preparations are prepared by known methods or usually usable prescriptions. For example, ointments are prepared by levigating or dissolving active ingredients in a base. The ointment base is selected from known or usually usable bases. For example, higher aliphatic acid or higher aliphatic acid ester (*e.g.*, adipic acid, myristic acid, palmitic acid, stearic acid, oleic acid, adipic acid ester, myristic acid ester, palmitic acid ester, stearic acid ester, oleic acid ester *etc.*), wax (*e.g.,* beeswax, whale wax, ceresin *etc.*), a surfactant (*e.g.*, polyoxyethylenealkylether phosphoric acid ester *etc.*), higher alcohol (*e.g.*, cetanol, stearyl alcohol, cetostearyl alcohol *etc.*), silicon oil (*e.g.,* dimethyl polysiloxane *etc.*), hydrocarbon (*e.g.*, hydrophilic petrolatum, white petrolatum, purified lanolin, liquid paraffin *etc.*), glycol (*e.g.*, ethylene glycol, diethylene glycol, propylene glycol, polyethylene glycol, macrogol *etc.*), vegetable oil (*e.g.*, castor oil, olive oil, sesame oil, turpentine oil), animal oil (mink oil, vitelline oil, squalane, squalene), water, an absorption accelerator and a rash preventive can be used singly or in combination of two or more thereof. The base can further contain a humectant, a preservative, a stabilizer, an antioxidant, a flavoring agent, *etc.*

The gels are prepared by, for example, dissolving active ingredients in a base. The gel base is selected from known or usually usable bases. For example, lower alcohol (*e.g.*, ethanol, isopropyl alcohol *etc.*), a gelling agent (*e.g.*, carboxymethyl cellulose, hydroxyethyl cellulose, hydroxypropyl cellulose, ethyl cellulose *etc.*), a neutralizing agent (*e.g.*, triethanolamine, diisopropanolamine *etc.*), a surfactant (*e.g.*, polyethylene glycol monostearate *etc.*), gums, water, an absorption accelerator, and a rash preventive are used singly or in combination of two or more thereof. The gel base can further contain a preservative, an antioxidant, a flavoring agent, *etc.*

The creams are prepared by, for example, dissolving or emulsifying active ingredients in a base. The cream base is selected from known or usually usable bases. For example, higher aliphatic acid ester, lower alcohol, hydrocarbon, polyvalent alcohol (*e.g.*, propylene glycol, 1,3-butylene glycol *etc.*), higher alcohol (*e.g.*, 2-hexyl decanol, cetanol *etc.*), emulsifier (*e.g.*, polyoxyethylene alkyl ethers, aliphatic acid esters *etc.*), water, an absorption accelerator, and a rash preventive are used singly or in combination of two or more thereof. The cream base can further contain a preservative, an antioxidant, a flavoring agent, *etc.*

The fomentations are prepared by, for example, dissolving active ingredients in a base to obtain a kneaded mixture, followed by spreading and applying it on a support. The fomentation base is selected from known or usually usable bases. For example, a thickener (*e.g.*, polyacrylic acid, polyvinyl pyrrolidone, gum arabic, starch, gelatin, methyl cellulose *etc.*), a wetting agent (*e.g.*, urea, glycerin, propylene glycol *etc.*), a filler (*e.g.,* kaolin, zinc oxide, talc, calcium, magnesium *etc.*), water, a solubilizing agent, a tackifier, and a rash preventive can be used singly or in combination of two or more thereof. The fomentations can further contain a preservative, an antioxidant, a flavoring agent, *etc.*

The cataplasms are prepared by, for example, dissolving active ingredients in a base to obtain a kneaded mixture, followed by spreading and applying it on a support. The cataplasm base is selected from known or usually usable bases. For example, a polymer base, fat, higher aliphatic acid, a tackifier and a rash preventive can be used singly or in combination of two or more thereof. The cataplasm base can further contain a preservative, an antioxidant, a flavoring agent, *etc.*

The liniments are prepared by, for example, dissolving, suspending or emulsifying active ingredients in water, alcohol (*e.g.*, ethanol, polyethylene glycol *etc.*), higher aliphatic acid, glycerin, soap, an emulsifier, a suspending agent, *etc.*, singly or in combination of two or more thereof. The liniment can further contain a preservative, an antioxidant, a flavoring agent, *etc.*

The nebulas and sprays are prepared by known and usually usable prescriptions. For example, they can comprise a usually usable diluent, additionally, a stabilizer such as sodium hydrogen sulfite, a buffer capable of providing isotonicity and a tonicity agent (e.g., sodium chloride, sodium citrate, or citric acid *etc.*).

The inhalants include aerosols, powders for inhalation and liquids for inhalation. The liquids for inhalation can be in the form which is used to be dissolved or suspended to water or other suitable vehicles just before use. For example,

the liquids for inhalation can contain an antiseptic (*e.g.* benzalkonium chloride, paraben *etc.*), a colorant, a buffer (*e.g.* sodium phosphate, sodium acetate *etc.*), a tonicity agent (*e.g.* sodium chloride, concentrated glycerin *etc.*), a thickener (*e.g.* carboxyvinylpolymer *etc.*), an absorption accelerator and so on, if necessary. The powders for inhalation can contain a lubricant (*e.g.* stearic acid and a salt thereof *etc.*), a binder (*e.g.* starch, dextrin *etc.*), an excipient (*e.g.* lactose, cellulose *etc.),* a colorant, an antiseptic (*e.g.* benzalkonium chloride, parlaben *etc.*), an absorption accelerator and so on. In order to administer the liquid for inhalation, a sprayer (*e.g.*, atomizer, nebulizer *etc.*) is normally used. In order to administer the powder for inhalation, a powder inhaler is normally used.

The injections for parenteral administration can be in the form of a solution, a suspension, an emulsion or a solid to be dissolved or suspended in a solvent just before use. The injections are prepared by, for example, dissolving, suspending or emulsifying active ingredients in a solvent. As such a solvent there can be used distilled water for injection, physiological saline, vegetable oil, alcohol such as propylene glycol, polyethylene glycol or ethanol, etc., singly or in combination thereof. The injections can further contain a stabilizer, a solubilizing agent (*e.g.*, glutamic acid, aspartic acid, Polysolvate 80 (trade name) *etc.*), a suspending agent, an emulsifier, a soothing agent, a buffer, a preservative, *etc.* The injections are sterilized at the final step or prepared by an aseptic process. Alternatively, an aseptic solid such as freeze-dried product which has previously been prepared can be dissolved in sterilized or aseptic distilled water for injection or other solvents just before use.

In case of using therapeutic agent for eye itching, preferred administration forms are eye drops, ophthalmic ointments, tablets and so on, more preferably eye drops or ophthalmic ointments. These can be prepared using general methods. For example, in case of eye drops, a tonicity agent, a buffer, a pH adjusting agent, a solubilizer, a thickener, a stabilizer, a preservative and so on can be added as additives. By adding a pH adjusting agent, a thickener, a dispersant and so on and suspending drugs, the stable eye drops can be obtained.

Tonicity agents include, for example, glycerin, propylenegrycol, sodium chloride, potassium chloride, sorbitol, mannitol and so on.

Buffers include, for example, phosphoric acid, phosphoric acid salt, citric acid, acetic acid, epsilon-aminocaproic acid and so on.

pH adjusting agents include, for example, hydrochloric acid, citric acid, phosphoric acid, acetic acid, sodium hydroxide, potassium hydroxide, boric acid, borax, sodium carbonate, sodium hydrogencarbonate and so on.

Solubilizers, for example, polysorbate 80, polyoxyethylene hydrogenated castor oil 60, macrogol 4000 and so on.

Thickeners and dispersants include, for example, cellulose polymers, such as hydroxypropylmethylcellulose, hydroxypropylcellulose and so on, polyvinyl alcohol, polyvinyl pyrrolidone and so on. Stabilizers include edetic acid, sodium edetate and so on.

Preservatives (antiseptics) include, for example, widely used sorbic acid, potassium sorbate, benzalkonium chloride, benzethonium chloride, methyl parahydroxybenzoate, propyl parahydroxybenzoate, chlorobutanol and so on. These can be combined to use.

The eye drops comprising the therapeutic agent for pruritus of the present invention are preferably adjusted to pH 4.0 to 8.5 and osmotic pressure ratio of about 1.0.

In case of using the therapeutic agent for pruritus, the dose of active ingredient can appropriately be selected depending on symptom, age of patients, forms of agents and so on. In case of oral preparations, they can be administered once or several times (*e.g.* 1 to 3 time(s)) per day each in an amount of preferably from 1mg to 100mg, more preferably from 5mg to 30mg. In case of eye drops, they can be administered one or several drops per one time, once or several times (*e.g.* 1 to 8 time(s)) per day, in concentration of preferably from 0.001 to 10% (w/v), more preferably from 0.01 to 1% (w/v). In case of ophthalmic ointments, they can be applied once or several times (*e.g.* 1 to 4 time(s)) per day in concentration of preferably from 0.001 to 10% (w/v), more preferably from 0.01 to 1% (w/v).

It goes without saying that the dose of these compounds can be less than the above-mentioned dose or sometimes it needs to exceed the above-mentioned range because the dose varies under various conditions as mentioned above.

As is apparent from the results of the after-mentioned pharmacological test, piperidine derivatives represented by formula (I) achieve an excellent itching inhibitory effect on eye itching. Therefore, piperidine derivatives of the present invention are useful as a therapeutic agent for itching of any types such as eye itching, skin itching, systemic itching and so on.

## BEST MODE FOR CARRYING OUT THE INVENTION

The pharmacological tests and preparation examples are described below, however, these examples are described in order to understand the present invention better and do not limit scope of the present invention.

[Pharmacological test]

The inhibitory effect on eye itching of piperidine derivatives of the present invention was studied using albumin induced eye itching model.

1. The test of inhibitory effect on eye itching using albumin induced eye itching model

(Experimental method)

Aluminum hydroxide gel absorption ovalbumin (20μg/mL)was dissolved in physiological saline solution, each solution (10mL) was injected subconjunctivally to the both eyeballs of five-week-old male Hartley guinea pigs to sensitize them actively. 14 days after sensitization, 1.0% (W/V) of ovalbumin physiological saline solution was instilled into both their eyes, 10μL/eye, respectively.

As test compounds, a suspension of 0.01% (W/V) and 0.1% (W/V) of {4-cyano-4-[3-(cyclopentyloxy)-4-(difluoromethoxy)phenyl]piperidine-1-yl}acetic acid monohydrate (hereinafter, it is the compound A) in 0.1% hydroxypropylmethylcellulose was prepared and it was instilled into both eyes of the above-mentioned guinea pigs, 10μL/eye, respectively, 30 minutes before the instillation of ovalbumin. 0.1% hydroxypropylmethylcellulose was used as control.

The behavior of guinea pigs for 60 minutes after ovalbumin instillation was video recorded. The inhibitory effect on eye itching was evaluated by counting each number of eye scratching times in case of instillation of each test compound solution and control. Table 1 shows eye scratching behavior inhibition ratio (average) to control, calculated according to the following equation. In addition, the number of example is eight.

$$\text{eye scratching behavior inhibition ratio} = 100 - ([\text{the number of eye scratching times of the test compound}] \div [\text{the number of eye scratching times of control}]) \times 100$$

Table 1

| test compounds | number of eye scratching times (times) | eye scratching behavior inhibition ratio (%) |
|---|---|---|
| Compound A (0.1%) | 29.0 | 57.4 |
| Compound A (0.01%) | 44.6 | 34.4 |
| Control | 68.0 | - |

(Experimental result)

As is apparent from table 1, the number of eye scratching times in the guinea pigs, to which the compound A: {4-cyano-4-[3-(cyclopentyloxy)-4-(difluoromethoxy)phenyl]piperidine-1-yl}acetic acid monohydrate was administered, decreases significantly compared to control. The extent almost depends on the concentration of the test compounds. As the above-mentioned result, it was noted that piperidine derivatives represented by formula (I) exhibit an excellent antipruritic effect.

[Preparation example]

The typical preparation examples according to in the present invention are shown below.

1. Eye drops

The eye drops prescribed below are prepared using general methods.

Prescription example 1

In 100mL,

| | |
|---|---|
| compound A | 100mg |

Table continued

| | |
|---|---|
| concentrated glycerin | 500mg |
| polysorbate 80 | 200mg |
| sodium dihydrogen phosphate dihydrate | q.s. |
| 1N sodium hydroxide | q.s. |
| hydrochloric acid | q.s. |
| sterile purified water | q.s. |

Eye drops comprising 10mg, 50mg, 100mg of the compound A in 100mL can be prepared in the same manner as Prescription example 1. In addition, the other piperidine derivatives of the present invention can be used instead of the compound A.

2. Ophthalmic ointment

The ophthalmic ointment prescribed below is prepared using general methods.

Prescription example 2

In 100g,

| | |
|---|---|
| compound A | 60mg |
| liquid paraffin | 10g |
| white petrolatum | q.s. |

Ophthalmic ointments having different concentrations can be prepared by appropriately changing the amount of the compound A in the same manner as Prescription example 2.

Industrial applicability

A piperidine derivative of the present invention is useful as a therapeutic agent for itching of any types such as eye itching, skin itching and systemic itching.

## Claims

1. Therapeutic agent for pruritus comprising a compound represented by formula (I)

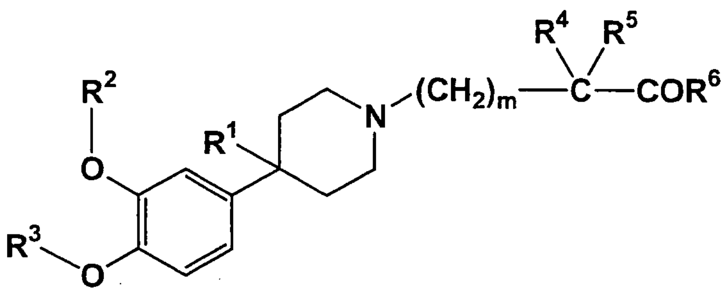

(I)

(wherein, $R^1$ is 1) a hydrogen atom, or 2) cyano, $R^2$ and $R^3$ are each independently 1) C1-8 alkyl, 2) C3-7 cycloalkyl, 3) C1-8 alkyl substituted by C3-7 cycloalkyl, 4) C1-8 alkyl substituted by 1, 2 or 3 halogen atom(s), 5) a hydrogen atom, 6) C1-8 alkyl substituted by phenyl, 7) C1-8 alkyl substituted by C1-8 alkoxy, or 8)

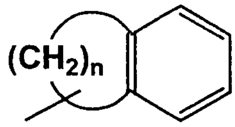

(wherein, n is 1 to 5.), $R^4$ and $R^5$ are each independently 1) a hydrogen atom, or 2) C1-8 alkyl, or $R^4$ and $R^5$ form a C3-7 saturated carbocyclic ring together with their binding carbon atom, $R^6$ is 1) hydroxyl, 2) C1-8 alkoxy, 3) -NHOH, or 4) C1-8 alkoxy substituted by phenyl, m is 0 or an integer of 1 to 4.),
a salt thereof, or a solvate thereof as the active ingredient,

**2.** The therapeutic agent for pruritus according to claim 1, wherein the compound is {4-cyano-4-[3-(cyclopentyloxy)-4-(difluoromethoxy)phenyl]piperidine-1-yl}acetic acid,

**3.** The therapeutic agent for pruritus according to claim 2, wherein the active ingredient is {4-cyano-4-[3-(cyclopentyloxy)-4-(difluoromethoxy)phenyl]piperidine-1-yl}acetic acid monohydrate,

**4.** The therapeutic agent for pruritus according to claim1, wherein the pruritus is eye itching,

**5.** The therapeutic agent for pruritus according to claim 1, wherein the therapeutic agent is in a form of a liquid preparation,

**6.** The therapeutic agent for pruritus according to claim 1, wherein the therapeutic agent is in a form of an external preparation,

**7.** The therapeutic agent for pruritus according to claim 5, wherein the liquid preparation is an eye drop,

**8.** The therapeutic agent for pruritus according to claim 6, wherein the external preparation is an ointment,

**9.** The therapeutic agent for pruritus according to claim 8, wherein the ointment is an ophthalmic ointment,

**10.** The therapeutic agent for pruritus according to claim 7, wherein the eye drop contains the active ingredient of 0.01 to 1 % (w/v),

**11.** A pharmaceutical composition comprising the therapeutic agent for pruritus according to claim 1 and at least one selected from steroidal anti-inflammatory drugs, nonsteroidal anti-inflammatory drugs, antiallergic drugs, antihistamine drugs, therapeutic agents for glaucoma, antibiotics, antibacterial drugs, antivirus drugs, and antifungal drugs,

**12.** A method for treating pruritus comprising administering to a mammal an effective amount of the compound represented by formula (I) described in claim 1, a salt thereof or a solvate thereof,

**13.** Use of the compound represented by formula (I) described in claim 1, a salt thereof or a solvate thereof for manufacturing a therapeutic agent for pruritus.

**INTERNATIONAL SEARCH REPORT**

International application No.
PCT/JP2004/010542

A. CLASSIFICATION OF SUBJECT MATTER
Int.Cl[7] A61K31/451, 9/06, 9/08, 45/00, A61P17/04, 27/02//C07D211/64

According to International Patent Classification (IPC) or to both national classification and IPC

B. FIELDS SEARCHED

Minimum documentation searched (classification system followed by classification symbols)
Int.Cl[7] A61K31/451, 9/06, 9/08, 45/00, A61P17/04, 27/02, C07D211/64

Documentation searched other than minimum documentation to the extent that such documents are included in the fields searched

Electronic data base consulted during the international search (name of data base and, where practicable, search terms used)
REGISTRY(STN), CAPLUS(STN)

C. DOCUMENTS CONSIDERED TO BE RELEVANT

| Category* | Citation of document, with indication, where appropriate, of the relevant passages | Relevant to claim No. |
|---|---|---|
| Y | WO 02/14280 A1 (Ono Pharmaceutical Co., Ltd.), 21 February, 2002 (21.02.02), Full text & AU 2001077738 A & EP 1308440 A1 & BR 2001013167 A & NO 2003000639 A & US 2004/044036 A1 | 1-11,13 |
| Y | JP 2003-89637 A (Nikken Chemicals Co., Ltd.), 28 March, 2003 (28.03.03), Full text; particularly, Par. Nos. [0046] to [0050] & WO 01/06000 A1 & EP 1410795 A1 | 1-11,13 |

[X] Further documents are listed in the continuation of Box C. [ ] See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 26 October, 2004 (26.10.04) | 16 November, 2004 (16.11.04) |
| Name and mailing address of the ISA/ Japanese Patent Office | Authorized officer |
| Facsimile No. | Telephone No. |

Form PCT/ISA/210 (second sheet) (January 2004)

**INTERNATIONAL SEARCH REPORT**

International application No.
PCT/JP2004/010542

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2002-179572 A (Nikken Chemicals Co., Ltd.), 26 June, 2002 (26.06.02), Full text; particularly, Par. Nos. [0044] to [0048] (Family: none) | 1-11,13 |
| Y | JP 2001-520196 A (SmithKline Beecham Corp.), 30 October, 2001 (30.10.01), Full text & WO 99/20280 A1 & ZA 9809450 A & CA 2306985 A & NO 20001847 A & BR 9814080 A & PL 341062 A & NZ 503551 A & CZ 20001376 A | 1-11,13 |
| Y | SCHMIDT, Bernhard M.W. et al., The phosphodiesterase 4 inhibitor roflumilast is effective in the treatment of allergic rhinitis, Journal of Allergy and Clinical Immunology, 2001, Vol.108, No.4, pages 530 to 536; full text | 1-11,13 |

Form PCT/ISA/210 (continuation of second sheet) (January 2004)

**INTERNATIONAL SEARCH REPORT**

International application No.
PCT/JP2004/010542

**Box No. II Observations where certain claims were found unsearchable (Continuation of item 2 of first sheet)**

This international search report has not been established in respect of certain claims under Article 17(2)(a) for the following reasons:

1. [X] Claims Nos.: 12
because they relate to subject matter not required to be searched by this Authority, namely:
Claim 12 pertains to methods for treatment of the human body by therapy.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**
[ ] The additional search fees were accompanied by the applicant's protest.
[ ] No protest accompanied the payment of additional search fees.

Form PCT/ISA/210 (continuation of first sheet (2)) (January 2004)